# EUROPEAN PATENT APPLICATION

(11) **EP 1 939 173 A1**
(43) Date of publication of application: **02.07.2008**
(21) Application number: 06026533.7
(22) Date of filing: 21.12.2006
(51) Int. Cl.: C07C 275/24, C07C 335/12, A61K 31/17, A61P 29/00

(54) **O-substituted-dibenzyl urea- or thiourea- derivatives as trpv1 receptor antagonists**

(71) Applicant: Pharmeste S.r.l., 44100 Ferrara (IT)
(72) Inventor: Baraldi, Pier Giovanni, 44100 Ferrara (IT); Borea, Pier Andrea, 44100 Ferrara (IT); Geppetti, Pierangelo, 44100 Ferrara (IT); Fruttarolo, Francesca, 44100 Ferrara (IT); Pavani, Maria Giovanna, 44100 Ferrara (IT); Trevisani, Marcello, 44100 Ferrara (IT)
(74) Representative: Minoja, Fabrizio

(57) **Abstract**

The invention relates to compounds of formula (I) 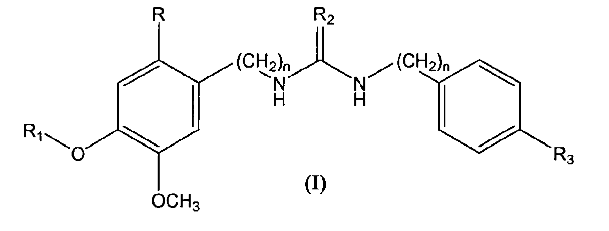 wherein
R is halogen;
R₁ is selected from 2-hydroxyethyl, 2,3-dihydroxypropyl, 3-hydroxypropyl, 2,2-dihydroxyethyl, 3,3-dihydroxypropyl, aminomethyl, 2-aminoethyl, 3-aminopropyl, 3-amino-2-hydroxypropyl, 1,3-dioxolane-ethyl, 1,3-dioxane-methyl, 1,3-dioxolane-methyl, 1,3-dioxane-ethyl, 3-fluoro-2-hydroxypropyl, 3-chloro-2-hydroxypropyl, 2-hydroxypropyl, 2-hydroxypropen-2-yl, morpholinoethyl, piperazinoethyl, hydroxymethyl, benzyl, 4-(hydroxymethyl)benzyl, 4-chlorobenzyl, 4-fluorobenzyl, 4-hydroxybenzyl, and 4-aminobenzyl;
R₂ is O or S;
R₃ is tert-butyl or trifluoromethyl;
n is an integer selected from 0, 1 or 2.

The compounds of formula (I) can be used for the preparation of pharmaceutical compositions for the therapy of inflammatory states, such as chronic neuropathic pain, over-active bladder syndrome, tumor pain, haemorrhoids, inflammatory hyperalgesia, post-intervention pain, dental extraction, airway and gastro-intestinal diseases.

## Description

### Field of the invention

The present invention relates to vanilloid receptor antagonists, in particular to O-hydroxyalkyl dibenzyl urea- or thiourea derivatives that antagonize the vanilloid TRPV1 receptor.

### State of the art

Recent experimental evidences have demonstrated that the expression of the vanilloid TRPV1 receptor (transient receptor potential channel) increases in the course of inflammatory states. This suggested that vanilloid receptor antagonists could be useful for the treatment of inflammatory pain states, for example chronic neuropathic pain, over-active bladder syndrome, haemorrhoids, inflammatory hyperalgesia, post-intervention pain, dental extraction, airway and gastro-intestinal diseases.

A number of vanilloid receptor antagonists are known; some of them derive from capsaicin and are referred to as capsaicinoid antagonists.

### Description of the invention

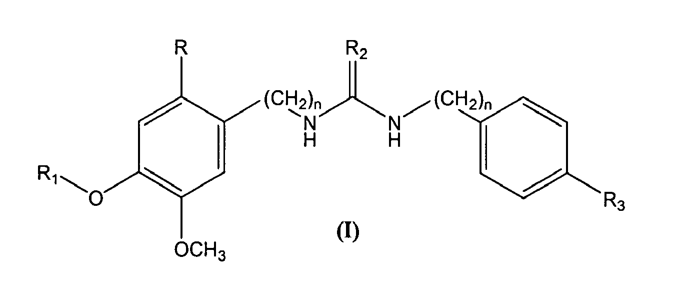

The present invention relates to compounds of formula (I) wherein:
R is halogen;
R₁ is selected from 2-hydroxyethyl, 2,3-dihydroxypropyl, 3-hydroxypropyl, 2,2-dihydroxyethyl, 3,3-dihydroxypropyl, aminomethyl, 2-aminoethyl, 3-aminopropyl, 3-amino-2-hydroxypropyl, 1,3-dioxolane-ethyl, 1,3-dioxane-methyl, 1,3-dioxolane-methyl, 1,3-dioxane-ethyl, 3-fluoro-2-hydroxypropyl, 3-chloro-2-hydroxypropyl, 2-hydroxypropyl, 2-hydroxypropen-2-yl, morpholinoethyl, piperazinoethyl, hydroxymethyl, benzyl, 4-(hydroxymethyl)benzyl, 4-chlorobenzyl, 4-fluorobenzyl, 4-hydroxybenzyl, and 4-aminobenzyl;
R₂ is O or S;
R₃ is *tert*-butyl or trifluoromethyl;
n is an integer selected from 0, 1 or 2.

The term "halogen" indicates a halogen selected from fluorine, chlorine, bromine or iodine.

A first preferred group of compounds of formula (I) is that wherein:
R is chlorine or bromine;
R₁ is 2-hydroxyethyl;
R₂ is S;
R₃ is tert-butyl or trifluoromethyl;
n is 1.

A second group of preferred compounds of formula (I) is that wherein:
R is chlorine or bromine;
R₁ is 2,3-dihydroxypropyl;
R₂ is S or O;
R₃ is tert-butyl or trifluoromethyl;
n is 1.

A third group of preferred compounds of formula (I) is that wherein:
R is chlorine or bromine;
R₁ is 2-hydroxyethyl;
R₂ is O;
R₃ is tert-butyl or trifluoromethyl;
n is 1.

A fourth group of preferred compounds of formula (**I**) is that wherein:
R is chlorine or bromine;
R₁ is 2,3-dihydroxypropyl;
R₂ is O;
R₃ is tert-butyl or trifluoromethyl;
n is 1.

Examples of particularly preferred compounds are:
1-[4-(2-hydroxyethoxy)-2-bromo-5-methoxybenzyl]-3-[4-(trifluoromethyl)-benzyl] thiourea;
1-[4-(2-hydroxyethoxy)-2-bromo-5-methoxybenzyl]-3-[4-(tert-butyl)-benzyl] thiourea;
1-[4-(2,3-dihydroxypropoxy)-2-bromo-5-methoxybenzyl]-3-[4-(trifluoromethyl)-benzyl] thiourea;
1-[4-(2,3-dihydroxypropoxy)-2-bromo-5-methoxybenzyl]-3-[4-(tert-butyl)-benzyl] thiourea;
1-[4-(2-hydroxyethoxy)-2-bromo-5-methoxybenzyl]-3-[4-(trifluoromethyl)-benzyl] urea;
1-[4-(2,3-dihydroxypropoxy)-2-chloro-5-methoxybenzyl]-3-[4-(trifluoromethyl)-benzyl] urea;
1-[4-(2-hydroxyethoxy)-2-bromo-5-methoxybenzyl]-3-[4-(*tert*-butyl)-benzyl] urea;
1-[4-(2,3-dihydroxypropoxy)-2-bromo-5-methoxybenzyl]-3-[4-(trifluoromethyl)-benzyl] urea;

The compounds of general formula (I) can be prepared by means of conventional methods, such as the reaction of a compound of formula (II), in which R and R₁ are as defined above, 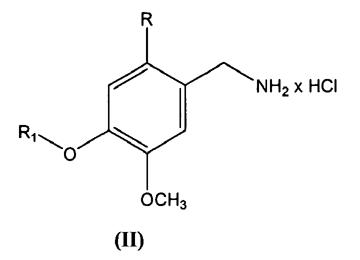 with a compound of formula (**III**) in which R₂ and R₃ are as defined above:

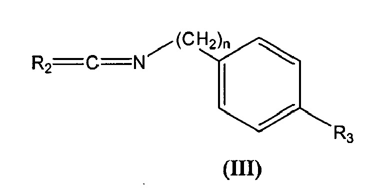

The compounds of formula (**I**) are able to inhibit the vanilloid TRPV1 receptor and can be used for the preparation of pharmaceutical compositions for the treatment of inflammatory states, chronic neuropathic pain, over-active bladder syndrome, haemorrhoids, inflammatory hyperalgesia, post-intervention pain, dental extraction, airway and gastro-intestinal diseases and tumour pain.

These formulations can be prepared by conventional methods and excipients, such as those disclosed in Remington's Pharmaceutical Sciences Handbook, XVII ed. Mack Pub., N.Y., USA.

The invention is hereinafter illustrated in greater detail in **Scheme 1** and in the **Examples**.

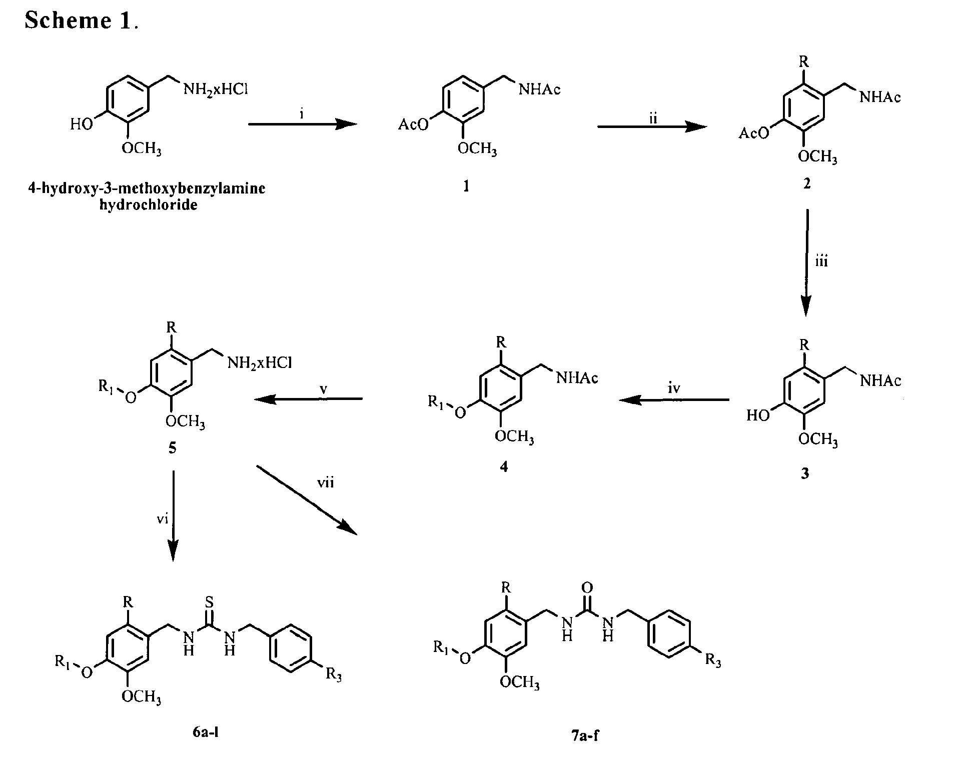

**Reagents and conditions**: (i) Acetic anidride, Pyr; (ii) NBS or NCS, DMF, O°C; (iii) aq. 10% HCl, Dioxane; (iv) hydroxyalkyl halide, K₂CO₃, DMF, 100°C; (v) HCl 37%, EtOH, Rfx; (vi) 4-(substituted)benzyl isothiocyanate, EtOH, Rfx; (vii) Triphosgene, 4-(substituted)benzyl amine, DIEA, CH₂Cl₂, 10 min.

**Substituents: 6a:** R= Cl, R₁=2-hydroxyethyl; R₃= tert-butyl; **6b:** R= Br, R₁=2-hydroxyethyl; R₃= *tert*-butyl; **6c:** R= Cl, R₁=2,3-dihydroxypropyl; R₃= trifluoromethyl; **6d:** R= Br, R₁=2,3-dihydroxypropyl; R₃= trifluoromethyl; **6e:** R= Cl, R₁= 3-hydroxypropyl; R₃= trifluoromethyl; **6f:** R= C1, R₁= 3-hydroxypropyl; R₃= tert-butyl; **6g:** R= Cl, R₁= 2-hydroxyethyl; R₃= trifluoromethyl; **6h:** R= Br, R₁= 2-hydroxyethyl; R₃= trifluoromethyl; **6i:** R= Cl, R₁= 2,3-dihydroxypropyl; R₃= *tert*-butyl; **6l:** R= Br, R₁= 2,3-dihydroxypropyl; R₃= tert-butyl; **7a**: R= Br, R₁= 2-hydroxyethyl; R₃= trifluoromethyl; **7b**: R= Cl, R₁= 2,3-dihydroxypropyl; R₃= tert-butyl; 7c: R= C1, R₁= 2-hydroxyethyl; R₃= tert-butyl; **7d**: R= Br, R₁= 2,3-dihydroxypropyl; R₃= trifluoromethyl; 7e: R= C1, R₁= 2,3-dihydroxypropyl; R₃= trifluoromethyl; **7f**: R= Br, R₁= 2-hydroxyethyl; R₃= *tert*-butyl.

### EXAMPLES:

The reactions were routinely monitored by thin-layer chromatography (TLC) on silica gel (precoated F₂₄₅ Merck plates) and the products were visualized with an iodine or potassium permanganate solution. ¹H NMR spectra were recorded in CDCl₃, CF₃COOD or DMSO-d₆ with a Varian VXR 200 spectrometer. Peak positions are given in parts per million (δ) downfield from tetramethylsilane as internal standard, and J values are given in Hz. IR spectra were recorded on a Pye Unicam SP 300 spectrometer using the KBr Wafer technique. Mass spectra were obtained with a Shimadzu QP5050 DI 50 spectrometer. The expression "Light petroleum ether" refers to the petroleum fraction boiling at 40-60°C. Melting points (M.p.) were determined on a Buchi-Tottoli instrument and are uncorrected. Chromatographies were performed using Merck 60-200 mesh silica gel. The synthesized compounds showed ¹H NMR spectra in agreement with the assigned structures. Elemental analyses were within ±0.4% of the theoretical values for C, H and N.

### Example 1. 1-[4-(2-hydroxyethoxy)-2-bromo-5-methoxybenzyl]-3-[4-(trifluoromethyl)-benzyl] thiourea (6h) and 1-[4-(2-hydroxyethoxy)-2-bromo-5-methoxybenzyl]-3-[4-(tert-butyl)-benzyl] thiourea (6b)

### 1.1. Synthesis of 4-acetoxy-3-methoxy-N-acetyl-benzylamine

Acetic anhydride (1 ml, 10.5 mmol) was added to a solution of 4-hydroxy-3-methoxy-benzylamine hydrochloride (0.5 g, 2.63 mmol) in pyridine (5 ml) and the mixture was stirred at room temperature for 6 hours. The solvent was evaporated off under reduced pressure and the residue was suspended in water (100 ml). The aqueous layer was extracted with ethyl acetate (3 x 20 ml) and the combined organic phases were anhydrified (Na₂SO₄) and evaporated under reduced pressure to afford the title compound as white solid (0.45 g, yield 75%).

¹H-NMR(CDCl₃) δ 2.01 (s, 3H, CH₃), 2.31 (s, 3H, CH₃), 3.81 (s, 3H, OCH₃), 4.38 (d, 2H, J=6, CH₂), 5.90 (bs, 1H, NH), 6.90 (m, 3H, aromatic).

MS: *m*/*z* 238.1 (M⁺ C₁₂H₁₅NO₄).

### 1.2. Synthesis of 2-bromo-4-acetoxy-5-methoxy-N-acetyl benzylamine

*N*-bromosuccinimide (6.3 mmol, 1.1 g) was added to a solution of 4-acetoxy-3-methoxy-N-acetyl-benzylamine of Example 1.1 (1.5 g, 4.2 mmol) in dry DMF (8 ml) and the mixture was stirred for 30' at 0°C and then for 16 hours at room temperature.

The formation of a white precipitate was observed when water (40 ml) was added to the reaction.

The solid was filtered off and washed twice with cold water (2 x 20 ml), then dried over P₂O₅ to afford the title compound as white solid (1.4 g, 99% yield).

¹H NMR (DMSO-d₆) δ 1.89 (s, 3H), 2.24 (s, 3H), 3.76 (s, 3H, OCH₃),4.27 (d, 2H, CH₂, J=8), 7.09 (s, 1H, aromatic), 7.25 (s, 1H, aromatic), 8.35 (t, 1H, NH).

Bidimensional NOESY (DMSO-d₆): coupling between the singlet at 2.24 ppm and the singlet at 7.25 ppm confirms that bromine is at the 2-position of the aromatic ring.

MS: m/z 316 (M⁺ C₁₂H₁₄BrNO₄).

### 1.3. Synthesis of 2-bromo-4-hydroxy-5-methoxy-N-acetyl benzylamine

10% Aq. hydrochloric acid (2.5 ml) was added to a solution of 2-bromo-4-acetoxy-5-methoxy-*N*-acetyl-benzylamine **2** (0.45 g, 1.66 mmol) in dioxane (15 ml) and the mixture was refluxed for 2 hours, then cooled and the solvent was concentrated under vacuum and the residue was basified with 10% aq. NaOH. The resulting solid was collected by filtration, washed with cold water and dried to furnish the title compound as white solid in quantitative yield.

¹H NMR (DMSO-d₆) δ 2.18 (s, 3H, CH₃), 3.87 (s, 3H, OCH₃), 4.00 (d, 2H, CH₂), 6.91 (s, 1H, aromatic), 7.32 (s, 1H, aromatic), 8.46 (t, 3H, NH₂), 9.80 (bs, 1H, OH).

M.p.: >300°C.

### 1.4. Synthesis of 2-bromo-4-(2-hydroxyethoxy)-5-methoxy-N-acetyl benzylamine

To a solution of compound **3** (0.4 g, 1.46 mmol) in dry DMF (15 ml) dry K₂CO₃ (2 mol eq) and 2-iodoethanol (2 mol eq) were added. The mixture was refluxed for 6 hours, then the solvent was evaporated off under reduced pressure. After addition of water the aqueous layer was extracted with EtOAc (3X 25 ml) and the organic phases were anhydrified over Na₂SO₄ and evaporated under reduced pressure to furnish the title compound as pale yellow solid (0.38 g, 81% yield).

¹H NMR (CDCl₃) δ 2.00 (s, 3H), 3.84 (s, 3H), 3.92 (t, 2H, J=2), 4.09 (t, 2H, J=2.1), 4.44 (d, 2H, J=4), 5.21 (t, 1H), 5.90 (bs, 1H), 6.96 (s, 1H), 7.07 (s, 1H).

### 1.5. Synthesis of 2-bromo-4-(2-hydroxyethoxy)-5-methoxybenzylamine hydrochloride

37% Hydrochloric acid (0.2 ml) was added to a solution of 2-bromo-4-(2-hydroxyethoxy)-5-methoxy-*N*-acetyl benzylamine **4** (0.1 g, 0.31 mmol) in abs. ethanol (5 ml) and the mixture was refluxed for 12 hours. After cooling, the solvent was evaporated off under reduced pressure and the residue was recrystallized from a methanol/ethyl ether mixture to afford the title compound as pale yellow solid in quantitative yield.

¹H NMR (DMSO-d₆) δ 3.95 (s, 3H), 4.14 (t, 2H, J=2), 4.19 (m, 2H,), 5.01 (m, 2H,), 5.44 (t, 1H), 7.02 (s, 1H), 7.27 (s, 1H), 7.38 (m, 3H).

### 1.6. 1-[4-(2-Hydroxyethoxy)-2-bromo-5-methoxybenzyl]-3-[4-(trifluoromethyl)-benzyl] thiourea 6h

TEA (1.2 mol eq) and 4-trifluoromethylbenzyl isothiocyanate (1.5 mol eq) were added to a suspension of amine hydrochloride 5 (0.3 mg, 0.9 mmol) in dry DMF (12 ml). The mixture was stirred at room temperature for 12 hours, then the solvent was evaporated off under reduced pressure and the residue was purified by flash chromatography (100% ethyl acetate) to afford the title compound as white solid (0.34 g, 74% yield).

¹H NMR (DMSO-d₆) δ 3.33 (s, 3H), 3.69 (m, 4H), 3.97 (t, 2H, J=2), 4.60 (bs, 2H), 4.74 (bs, 2H), 4.86 (t, 1H, J=2.1), 6.92 (bs, 1H), 7.16 (s, 1H), 7.51 (d, 2H, J=7.8), 7.70 (d, 2H, J=8), 7.96 (bs, 1H), 8.18 (bs, 1H).

Mp: 140-2°C.

MS: *m*/*z* 493.3 (M⁺ C₁₉H₂₀BrF₃N₂O₃S).

### 1.7. 1-[4-(2-Hydroxyethoxy)-2-bromo-5-methoxybenzyl]-3-[4-(tert-butyl)-benzyl] thiourea 6b

TEA (1.2 mol eq) and 4-*tert*-butylbenzyl isothiocyanate (1.5 mol eq) were added to a suspension of amine hydrochloride 5 (60 mg, 0.19 mmol) in dry DMF (10 ml). The mixture was stirred at room temperature for 12 hours, then the solvent was evaporated off under reduced pressure and the residue was purified by flash chromatography (100% ethyl acetate) to afford the title compound as pale pink solid (55 mg, 64% yield).

¹H NMR (DMSO-d₆) δ 1.26 (s, 9H), 3.37 (s, 3H), 3.70 (m, 4H), 3.98 (t, 2H, J=2), 4.61 (bs, 4H), 4.87 (t, 1H, J=2.1), 6.98 (bs, 1H), 7.21 (s, 1H), 7.23 (d, 2H, J=7.8), 7.34 (d, 2H, J=8), 7.80 (bs, 1H), 8.00 (bs, 1H).

Mp: 138°C.

MS: *m*/*z* 481.4 (M⁺ C₂₂H₂₉BrN₂O₃S).

### Example 2. 1-[4-(2,3-Dihydroxypropoxy)-2-bromo-5-methoxybenzyl]-3-[4-(trifluoromethyl)-benzyl] thiourea 6d and 1-[4-(2,3-dihydroxypropoxy)-2-bromo-5-methoxybenzyl]-3-[4-(tert-butyl)-benzyl] thiourea 61

### 2.1. Synthesis of 2-bromo-4-(2,3-dihydroxypropoxy)-5-methoxy-N-acetyl benzylamine

To a solution of 2-bromo-4-hydroxy-5-methoxy-*N*-acetyl benzylamine 3 (0.3 g, 1.1 mmol) in dry DMF (10 ml) dry K₂CO₃ (2 mol eq) and 3-chloro-1,2-dihydroxypropane (2 mol eq) were added. The mixture was refluxed for 6 hours, then the solvent was evaporated off under reduced pressure. After addition of water the aqueous layer was extracted with EtOAc (3X 25 ml) and the organic phases were anhydrified over Na₂SO₄ and evaporated off under reduced pressure to furnish the title compound as pale yellow solid (0.35 g, 84% yield).

¹H NMR (DMSO-d₆) δ 1.88 (s, 3H), 3.44 (t, 2H), 3.74 (s, 3H), 3.88-3.96 (m, 3H), 4.22 (d, 2H, J=6), 4.66 (t, 1H), 4.96 (d, 1H, J=6), 6.93 (s, 1H), 7.14 (s, 1H), 8.25 (t, 1H).

### 2.2. Synthesis of 2-bromo-4-(2,3-dihydroxypropoxy)-5-methoxybenzylamine hydrochloride

37% Hydrochloric acid (0.3 ml) was added to a solution of 2-bromo-4-(2,3-dihydroxypropoxy)-5-methoxy-N-acetyl benzylamine **4** (0.3 g, 0.86 mmol) in abs. ethanol (12 ml) and the mixture was refluxed for 12 hours. After cooling, the solvent was evaporated off under reduced pressure and the residue was recrystallized from a methanol/ethyl ether mixture to afford the title compound as pale orange solid in quantitative yield.

¹H NMR (DMSO-d₆) δ 3.42 (t, 2H), 3.74 (s, 3H), 3.74-3.95 (m, 4H), 4.21 (d, 2H, J=6), 4.98 (m, 4H), 7.13 (s, 1H), 7.38 (s, 1H).

### 2.3. Synthesis of 1-[4-(2,3-dihydroxypropoxy)-2-bromo-5-methoxybenzyl)-3-(4-(trifluoromethyl)-benzyl] thiourea 6d

TEA (2 mol eq) and 4-trifluoromethylbenzyl isothiocyanate (2.5 mol eq) were added to a suspension of 2-bromo-4-(2,3-dihydroxypropoxy)-5-methoxy-benzylamine hydrochloride (0.1 g, 0.29 mmol) in dry DMF (8 ml). The mixture was stirred at room temperature for 12 hours, then the solvent was evaporated off under reduced pressure and the residue was purified by flash chromatography (7/3 ethyl acetate/light petroleum) to afford the title compound as white solid (85 mg, 58% yield).

¹H NMR (DMSO-d₆) δ 3.42-3.53 (m, 3H), 3.80 (s, 3H), 4.06-4.13 (m, 4H), 4.67-4.75 (m, 4H), 6.18 (bs, 1H), 6.25 (bs, 1H), 7.01 (s, 1H), 7.03 (s, 1H), 7.40 (d, 2H, J=10), 7.59 (d, 2H, J=10).

Mp.: 145-6°C.

MS: *m*/*z* 523.36 (M⁺ C₂₀H₂₂BrF₃N₂O₄S).

### 2.4. Synthesis of 1-[4-(2,3-dihydroxypropoxy)-2-bromo-5-methoxybenzyl]-3-[4-(tert-butyl)-benzyl] thiourea 61

TEA (2 mol eq) and 4-*tert*-butylbenzyl isothiocyanate (2.5 mol eq) were added to a suspension of 2-bromo-4-(2,3-dihydroxypropoxy)-5-methoxybenzylamine hydrochloride (0.1 g, 0.29 mmol) in dry DMF (8 ml). The mixture was stirred at room temperature for 12 hours, then the solvent was evaporated off under reduced pressure and the residue was purified by flash chromatography (8/2 ethyl acetate/light petroleum) to afford the title compound as pale orange solid (90 mg, 61% yield).

¹H NMR (DMSO-d₆) δ 1.31 (s, 9H), 3.44-3.51 (m, 3H), 3.80 (s, 3H), 4.16-4.23 (m, 4H), 4.70-4.76 (m, 4H), 6.21 (bs, 1H), 6.27 (bs, 1H), 7.11 (s, 1H), 7.14 (s, 1H), 7.38 (d, 2H, J=10), 7.62 (d, 2H, J=9.8).

Mp: 152-3°C.

MS: m/z 511.4 (M⁺ C₂₃H₃₁BrN₂O₄S).

### Example 3. 1-(4-(2,3-Dihydroxypropoxy)-2-chloro-5-methoxybenzyl)-3-(4-(tert-butyl)-benzyl) thiourea 6i

### 3.1. Synthesis of 2-chloro-4-acetoxy-5-methoxy-N-acetyl benzylamine

*N*-chlorosuccinimide (3.15 mmol, 0.42 g) was added to a solution of 4-acetoxy-3-methoxy-*N*-acetyl-benzylamine of Example 1.1 (0.5 g, 2.1 mmol) in dry DMF (6 ml) and the mixture was stirred for 30' at 0°C and then for 16 hours at room temperature.

When water was added to the reaction (40 ml) the formation of a white precipitate was observed.

The solid was filtered off and washed twice with cold water (2 x 20 ml), then dried over P₂O₅ to afford the title compound as white solid (0.45 g, 83% yield).

¹H NMR (DMSO-d₆) δ 1.85 (s, 3H), 2.21 (s, 3H), 3.74 (s, 3H, OCH₃),4.21 (d, 2H, CH₂, J=8), 7.01 (s, 1H, aromatic), 7.22 (s, 1H, aromatic), 8.32 (t, 1H, NH).

Bidimensional NOESY (DMSO-d₆): coupling between the singlet at 2.21 ppm and the singlet at 7.22 ppm confirms that chlorine is at the 2-position of the aromatic ring.

MS: *m*/*z* 272.1 (M⁺ C₁₂H₁₄ClNO₄).

### 3.2. Synthesis of 2-chloro-4-hydroxy-5-methoxy-N-acetyl benzylamine

10% Aq. hydrochloric acid (2.5 ml) was added to a solution of 2-chloro-4-acetoxy-5-methoxy-N-acetyl-benzylamine 2 (0.45 g, 1.66 mmol) in dioxane (15 ml) and the mixture was refluxed for 2 hours. After cooling, the solvent was reduced under vacuum and the residue was basified with 10% aq. NaOH. The resulting solid was collected by filtration, washed with cold water and dried to furnish the title compound as white solid in quantitative yield.

¹H NMR (DMSO-d₆) δ 2.15 (s, 3H, CH₃), 3.82 (s, 3H, OCH₃), 3.99 (d, 2H, CH₂), 6.86 (s, 1H, aromatic), 7.30 (s, 1H, aromatic), 8.41 (t, 3H, NH₂), 9.77 (bs, 1H, OH).

M.p.: >300°C.

### 3.3. Synthesis of 2-chloro-4-(2,3-dihydroxypropoxy)-5-methoxy-N-acetyl benzylamine

Dry K₂CO₃ (2 mol eq) and 2-iodoethanol (2 mol eq) were added to a solution of 2-chloro-4-hydroxy-5-methoxy-N-acetyl benzylamine 3 (0.4 g, 1.46 mmol) in dry DMF (15 ml). The mixture was refluxed for 6 hours, then the solvent was evaporated off under reduced pressure. After addition of water the aqueous layer was extracted with EtOAc (3X 25 ml) and the organic phases were anhydrified over Na₂SO₄ and evaporated under reduced pressure to furnish the title compound as pale yellow solid (0.38 g, 81% yield).

¹H NMR (CDCl₃) δ 2.00 (s, 3H), 3.84 (s, 3H), 3.92 (t, 2H, J=2), 4.09 (t, 2H, J=2.1), 4.44 (d, 2H, J=4), 5.21 (t, 1H), 5.90 (bs, 1H), 6.96 (s, 1H), 7.07 (s, 1H).

### 3.4. Synthesis of 2-chloro-4-(2,3-dihydroxypropoxy)-5-methoxybenzylamine hydrochloride

37% Hydrochloric acid (0.2 ml) was added to a solution of 2-bromo-4-(2-hydroxyethoxy)-5-methoxy-N-acetyl benzylamine **4** (0.1 g, 0.31 mmol) in abs. ethanol (5 ml) and the mixture was refluxed for 12 hours. After cooling, the solvent was evaporated off under reduced pressure and the residue was recrystallized from a mixture of methanol/ethyl ether to afford the title compound as pale yellow solid in quantitative yield.

### 3.5. Synthesis of 1-[4-(2,3-dihydroxypropoxy)-2-chloro-5-methoxybenzyl]-3-[4-(tert-butyl)-benzyl] thiourea 6i

TEA (1.2 mol eq) and 4-*tert*-butylbenzyl isothiocyanate (2.5 mol eq) were added to a suspension of the amine hydrochloride from example 3.4 (0.1 g, 0.335 mmol) in dry DMF (8 ml). The mixture was stirred at room temperature for 12 hours, then the solvent was evaporated off under reduced pressure and the residue was purified by flash chromatography (100% ethyl acetate) to afford the title compound as pale pink solid (0.110 g, 70 % yield).

¹H NMR (DMSO-d₆) δ 1.28 (s, 9H), 3.32-3.49 (m, 3H), 3.74 (s, 3H), 4.10-4.20 (m, 4H), 4.61-4.75 (m, 4H), 6.17 (bs, 1H), 6.19 (bs, 1H), 7.03 (s, 1H), 7.05 (s, 1H), 7.31 (d, 2H, J=9.9), 7.58 (d, 2H, J=9.8).

Mp: 157°C.

MS: *m*/*z* 467 (M⁺ C₂₃H₃₁ClN₂O₄S).

### Example 4. General procedure for the synthesis of 1-[4-(2-hydroxyethoxy)-2-bromo-5-methoxybenzyl]-3-[4-(trifluoromethyl)-benzyl] urea 7a, 1-[4-(2,3-dihydroxypropoxy)-2-chloro-5-methoxybenzyl]-3-[4-(trifluoromethyl)-benzyl] urea 7e, 1-[4-(2-hydroxyethoxy)-2-bromo-5-methoxybenzyl]-3-[4-(tert-butyl)-benzyl] urea 7f and 1-[4-(2,3-dihydroxypropoxy)-2-bromo-5-methoxybenzyl]-3-[4-(trifluoromethyl)-benzyl] urea 7d

Triphosgene (0.37 mol eq) was dissolved in CH₂Cl₂ (3 ml). A mixture of 4-*tert*-butyl/trifluoromethyl benzyl amine (0.33 mmol) and DIEA (2.2 mol eq) in CH₂Cl₂ (2 ml) was slowly added to the stirred solution of triphosgene over a period of 30 min. using a syringe pump. After 5 min a solution of a suitable amine hydrochloride 5 (0.33 mmol) was added in one portion. The reaction mixture was stirred at room temperature for 2-4 h, evaporated under reduced pressure, diluted with EtOAc (20 ml), washed with 10% aq. KHSO₄, 5% aq. NaHCO₃ and brine, dried over Na₂SO₄ and evaporated to dryness. The residue was purified by flash chromatography (100% EtOAc) to furnish the title compound as solid.

### 4.1. 1-[4-(2-Hydroxyethoxy)-2-bromo-5-methoxybenzyl]-3-[4-(trifluoromethyl)-benzyl] urea 7a

White solid, yield 73%.

¹H NMR (DMSO-d₆) δ 3.67 (s, 3H), 3.47 (m, 2H), 3.94 (t, 2H, J=4), 4.16 (d, 2H, J=6), 4.32 (d, 2H, J=6), 4.85 (t, 1H, J=2), 6.52 (bt, 1H), 6.68 (bt, 1H), 6.90 (s, 1H), 7.14 (s, 1H), 7.49 (d, 2H, J=8), 7.65 (d, 2H, J=8).

MS: *m*/*z* 477 (M⁺ C₁₉H₂₀BrF₃N₂O₄).

Mp: 162°C.

### 4.2. 1-[4-(2,3-Dihydroxypropoxy)-2-chloro-5-methoxybenzyl]-3-[4-(trifluoromethyl)-benzyl] urea 7e

White solid, yield 80%.

¹H NMR (DMSO-d₆) δ 3.44 (t, 2H, J=6), 3.68 (s, 1H), 3.74-4.00 (m, 5H), 4.22 (d, 2H, J=6), 4.32 (d, 2H, J=6), 4.67 (t, 1H), 4.94 (d, 1H), 6.45 (bt, 1H), 6.65 (bt, 1H), 6.90 (s, 1H), 7.00 (s, 1H), 7.48 (d, 2H, J=7.8), 7.69 (d, 2H, J=8).

MS: *m*/*z* 462 (M⁺ C₂₀H₂₂ClF₃N₂O₅).

Mp: 154-5°C.

### 4.3. 1-[4-(2-Hydroxyethoxy)-2-bromo-5-methoxybenzyl]-3-[4-(tert-butyl)-benzyl] urea 7f

White solid, yield 78%.

¹H NMR (DMSO-d₆) δ 1.25 (s, 9H), 3.68 (m, 5H), 3.96 (t, 2H, J=2), 4.17 (m, 4H), 4.85 (t, 1H), 6.37 (t, 1H), 6.50 (t, 1H), 6.91 (s, 1H), 7.13 (s, 1H), 7.16 (d, 2H, J=7), 7.32 (d, 2H, J=8).

MS: *m*/*z* 465 (M⁺ C₂₂H₂₉BrN₂O₄).

Mp: 145-7°C.

### 4.4. 1-[4-(2,3-Dihydroxypropoxy)-2-bromo-5-methoxybenzyl]-3-[4-(trifluoromethyl)-benzyl] urea 7d

White solid, yield 84%.

¹H NMR (DMSO-d₆) δ 3.41 (t, 2H, J=6), 3.74 (s, 1H), 4.00-3.85 (m, 5H), 4.19 (d, 2H, J=6), 4.32 (d, 2H, J=6), 4.62 (t, 1H), 4.95 (d, 1H), 6.49 (bt, 1H), 6.68 (bt, 1H), 6.90 (s, 1H), 7.13 (s, 1H), 7.45 (d, 2H, J=7.8), 7.65 (d, 2H, J=8).

MS: *m*/*z* 507 (M⁺ C₂₀H₂₂BrF₃N₂O₅).

Mp: 164°C.

### Biological Assays

### Animals

*In vivo* experiments were conducted with PharmEste srl (Ferrara, Italy) and with the University of Ferrara, following protocols approved by the Animal Care and Use Committee of the University of Ferrara.

### Radioligand binding assay

Male Sprague-Dawley rats with body weight between 250 to 350 g were used. For binding assays the rats were decapitated under anesthesia and the spinal cord was removed and disrupted using a Polytron tissue homogenizer in ice cold buffer containing 5 mM KCl, 5.8 mM NaCl, 0.75 mM CaCl₂, 2 mM MgCl₂, 320 mM sucrose, 10 mM Hepes, pH 8.6 (Szallasi and Blunberg, 1992; 1993). In competition experiments, the membranes were incubated at 37°C for 60 min with [³H]RTX (0.4 nM) and with increasing concentrations of test compounds in the range from 0.1 nM to 3 µM. Non-specific binding was evaluated in the presence of 1 µM RTX.. Saturation and competition studies were analyzed with the Ligand program (Bradford, 1976; Munson and Rodbard, 1980).

### Ca²⁺ fluorescence measurements in cultured rat trigeminal ganglia

The calibration curve was determined using a buffer containing Fura-2-AM-ester and definite concentrations of free Ca²⁺. This curve was then used to convert the data obtained from F₃₄₀/F₃₈₀ ratio to [Ca²⁺]ᵢ (nM) (Kudo, Y). The effects of pretreatments with compounds **6b, 6d, 6i, 6h, 6l, 7a, 7e, 7d** and **7f** on the increase in [Ca²⁺]; produced by 0.1 µM capsaicin were studied.

### Capsaicin-induced secondary allodynia in rat

Capsaicin (20 nmols/50 µl/paw) was injected in the plantar surface of the glabrous skin of the right paw of rats anesthetized with diethyl ether (Chaplan et al., 1994). Compounds 6h and 7e were orally administrated 2 hours prior to capsaicin injection. Tactile allodynia was evaluated 90 min after capsaicin challenge.

### Reagents

The stock concentrations of capsaicin (10 mM) was prepared in absolute ethanol. Compounds **6b**, **6d**, **6i**, **6h**, **6l**, **7a**, **7e**, **7d** and **7f** were prepared in 50% DMSO and 50% Tween 80. Fura-2-AM-ester and ionomycin were dissolved in 100% DMSO. All the other drugs were dissolved in distilled water. The appropriate dilutions were then made in Krebs buffer solution.

### Results

### Radioligand binding assays

Compounds **6b, 6d, 6i, 6h, 61, 7a, 7e, 7d** and **7f** displaced [³H]RTX from its binding site in rat spinal cord membranes at low concentrations, as indicated by the K; values reported in Table 1.

### Ca²⁺ fluorescence Assay

Capsaicin (0.1 µM) increased [Ca²⁺]ᵢ in the vast majority (95%) of rat trigeminal neuron cells, which were therefore identified as TRPV1-expressing neurons. IC₅₀ values of inhibiting capsaicin-evoked [Ca²⁺]ᵢ mobilization are summarized in **Table 1**.

**Table 1: Kᵢ and IC₅₀ values of some representative compounds of the invention.**

| **Code** | **6i** | **6h** | **6b** | **6d** | **61** | **7e** | **7a** | **7f** | **7d** |
|---|---|---|---|---|---|---|---|---|---|
| **Kᵢ (nM)** | 48 | 75 | 60 | 27 | 20 | 60 | 150 | 76 | 86 |
| **IC₅₀ (nM)** | 39.7 | 81.1 | 56.8 | 9.7 | 31.8 | 10.2 | 119 | 61 | 80.9 |

The results are expressed as Mean and 95% fiducial limits.

### Capsaicin-induced secondary allodynia in rat

In a more extended study, compounds **6h** and **7e** were tested against capsaicin-induced secondary allodynia in rats. 90 Min after the capsaicin challenge, compounds **6h** and **7e** (both at 10 mg/kg, p.o), significantly prevented the pro-allodinic effect of capsaicin (53.1% and 47.9% of inhibition, respectively).

### ADME Studies

In order to select suitable drug candidates, ADME studies *in vitro* were performed on selected compounds **6h** and **7e**, so as to assess the properties of these compounds according to the substituents.

LogD Values at pH=7.0 were calculated in silico, while the *in vitro* tests analysed:
- metabolic stability in cryopreserved human hepatocytes;
- cytotoxicity on Hep G2 cells;
- cassette pharmacokinetics in rat.

The data of the compounds of the invention were compared to those obtained on two structurally different compounds recently disclosed as TRPV1 antagonists, namely JYL 1421 (Jakab et al., 2005) and SB-705498 (Rami et al., 2006) and to those obtained with two widely used drugs, one with short half life (naloxone) and one with long half-life (tolbutamide). The most relevant ADME data allow rapid comparison of the influence of specific substituents, especially on metabolic stability.

### Hepatocytes preparation

The cells were rapidly and carefully thawed and diluted in ice-cold Krebs-Henseleit Buffer (KHB). After centrifugation (50 g, 5 min.) the supernatant was discharged and the cells were resuspended in a volume of ice-cold KHB to a greater density than 2X (with respect to the final concentration of incubation) of viable cells/ml based on nominal concentration in cryopreserved vials. The viable cells were counted by Trypan Blue exclusion with a haemocytometer and the concentration of viable hepatocytes was accurately corrected to 2X concentration with KHB.

### Hep G2 cells preparation

The cells were cultured for 3 days, trypsinized and re-suspended in 20 ml of culture medium. The cells were then counted and diluted to obtain a final concentration suitable for seeding 40.000 cells/well in 96-well cell culture plates (200 µl/well).

The cells were seeded in columns 1 to 11 (column 12 contained medium without cells), then placed for 16-24 hours at 37°C with 5% of CO₂.

### Compounds preparation

Test and reference compounds were prepared at 2X incubation concentrations (10 and 1 µM) diluting 10 µl of stock solution in 0.99 ml of KHB to a concentration of 10 µM and 5 µl of stock solution in 0.995 µl of KHB to a concentration 5 µM. 300 µl of 10 µM and 1 µM solutions were then dispensed respectively in 2 and 1 incubation test tubes (Sterilin T.C. tube 17 x 100 mm).

### Preliminary cassette pharmacokinetic study in catheterized conscious rats

Compounds were administered together to rats. The compounds were stored at -20°C when not used. The formulation, route of administration, plasma samples identification and pharmacokinetic analysis were performed according to standard protocols (Raynaud, FI et al, 2004; Manitpisitkul, P. et al., 2004; Singh S. et al., 2006).

### Results

With respect to compounds **Ia, Ib, Ic** disclosed in WO 2005/123666 A1, this new series of O-hydroxyalkyl derivatives showed a clear unexpected improvement in terms of metabolic stability comparable to reference compounds JYL 1421 and SB-705498 and a good half-life time, their clearance being relatively slow. Also cytotoxicity values expressed as micromolar IC₅₀ were acceptable. **Table 2** reports the ADME profile of two compounds of the invention with respect to compounds disclosed in WO 2005/123666.

**Table 2. ADME profile of compounds 6h and 7e and selected reference compounds**

| **Code** | **Cytotoxicity in Hep G2 cells IC₅₀µM** | **Metabolic stability in Human Hepatocytes Clᵢₙₜ** | | **Pharmacokinetics in Rat T_{1/2} min** | | |
|---|---|---|---|---|---|---|
| ***Ia ^{Ref. 1}*** | 21.9 | 1.81 | | 19 | | |
| ***Ib ^{Ref.1}*** | 22.2 | 2.19 | | 14 | | |
| ***Ic^{Ref.1}*** | 18.3 | 2.21 | | 19 | | |
| ***JYL 1421*** | - | 0.9 | | Ref. 7 | | |
| ***SB-705498*** | 20.3 | 0.33 | | 180 ^{Ref.8} | | |
| ***Naloxone*** | - | 1.99-2.30 | | - | | |
| ***Tolbutamide*** | - | 0.04-0.38 | | - | | |
| **6h** | **52** | **0.4** | | **104** | | |
| **7e** | **70** | **0.6** | | **78** | | |

### References

**1. PharmEste S.r.l.** WO 2005/123666 A1.
**2.** Bradford MM. Anal Biochem. 1976, 72, 248-254.
**3.** Munson PJ. et al., Anal Biochem 1980, 107, 220-239.
**4.** Rigoni, M. et al., Br. J. Pharmacol. 2003, 138, 977-985.
**5.** Kudo, Y. et al., Jap. J. Pharmacol. 1986, 41, 345-351.
**6.** Chaplan N. et al., J Neurosci Methods. 1994, 53, 55-63
**7.** Jakab B. et al. Eur. J. Pharmacol. 2005, 517, 35-44.
**8.** Rami HK. et al. Bioorg. Med. Chem. Lett. 2006, 16, 3287-3291.
**9.** Raynaud FI, et al. Mol. Cancer Ther. 2004, 3, 353-362.
**10.** Manitpisitkul, P. et al. Drug Discov. Today, 2004, 9, 652-658.
**11.** Singh S. Curr. Drug Metab. 2006, 7, 165-182.

## Claims

1. Compounds of formula (I) 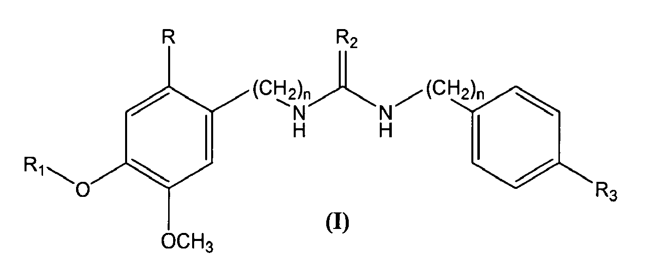 wherein
R is halogen;
R₁ is selected from 2-hydroxyethyl, 2,3-dihydroxypropyl, 3-hydroxypropyl, 2,2-dihydroxyethyl, 3,3-dihydroxypropyl, aminomethyl, 2-aminoethyl, 3-aminopropyl, 3-amino-2-hydroxypropyl, 1,3-dioxolane-ethyl, 1,3-dioxane-methyl, 1,3-dioxolane-methyl, 1,3-dioxane-ethyl, 3-fluoro-2-hydroxypropyl, 3-chloro-2-hydroxypropyl, 2-hydroxypropyl, 2-hydroxypropen-2-yl, morpholinoethyl, piperazinoethyl, hydroxymethyl, benzyl, 4-(hydroxymethyl)benzyl, 4-chlorobenzyl, 4-fluorobenzyl, 4-hydroxybenzyl, and 4-aminobenzyl;
R₂ is O or S;
R₃ is tert-butyl or trifluoromethyl;
n is an integer selected from 0, 1 or 2.

2. Compounds according to claim 1 wherein:
R is chlorine or bromine;
R₁ is 2-hydroxyethyl;
R₂ is S;
R₃ is tert-butyl or trifluoromethyl;
n is 1.

3. Compounds according to claim 1 wherein:
R is chlorine or bromine;
R₁ is 2,3-dihydroxypropyl;
R₂ is S;
R₃ is tert-butyl or trifluoromethyl;
n is 1.

4. Compounds according to claim 1 wherein:
R is chlorine or bromine;
R₁ is 2,3-dihydroxypropyl;
R₂ is O;
R₃ is *tert*-butyl or trifluoromethyl;
n is 1.

5. Compounds according to claim 1 wherein:
R is chlorine or bromine;
R₁ is 2-hydroxyethyl;
R₂ is O;
R₃ is tert-butyl or trifluoromethyl;
n is 1.

6. A compound selected from:
1-[4-(2-hydroxyethoxy)-2-bromo-5-methoxybenzyl]-3-[4-(trifluoromethyl)-benzyl] thiourea;
1-[4-(2-hydroxyethoxy)-2-bromo-5-methoxybenzyl]-3-[4-(*tert*-butyl)-benzyl] thiourea;
1-[4-(2,3-dihydroxypropoxy)-2-bromo-5-methoxybenzyl]-3-[4-(trifluoromethyl)-benzyl] thiourea;
1-[4-(2,3-dihydroxypropoxy)-2-bromo-5-methoxybenzyl]-3-[4-(tert-butyl)-benzyl] thiourea;
1-[4-(2-hydroxyethoxy)-2-bromo-5-methoxybenzyl]-3-[4-(trifluoromethyl)-benzyl] urea;
1-[4-(2,3-dihydroxypropoxy)-2-chloro-5-methoxybenzyl]-3-[4-(trifluoromethyl)-benzyl] urea;
1-[4-(2-hydroxyethoxy)-2-bromo-5-methoxybenzyl]-3-[4-(tert-butyl)-benzyl] urea;
1-[4-(2,3-dihydroxypropoxy)-2-bromo-5-methoxybenzyl]-3-[4-(trifluoromethyl)-benzyl] urea.

7. Compounds of formula (I) as defined in any one of claims 1 to 6 for use as medicaments.

8. Use of compounds of formula (I) as defined in any one of claims 1 to 6 as vanilloid TRPV1 receptor antagonists.

9. Use of compounds of formula (I) as defined in any one of claims 1 to 6 for the preparation of pharmaceutical compositions for the therapy of inflammatory states.

10. Use according to claim 9 wherein the inflammatory state is selected from chronic neuropathic pain, over-active bladder syndrome, tumour pain, haemorrhoids, inflammatory hyperalgesia, post-intervention pain, dental extraction, airway and gastro-intestinal diseases.

11. Pharmaceutical compositions containing compounds of formula (I) as defined in any one of claims 1 to 6 in admixture with suitable excipients and/or vehicles.
